**Europäisches Patentamt**

**European Patent Office**

**Office européen des brevets**

(19)

(11) Veröffentlichungsnummer: **0 001 570**
**B1**

(12) **EUROPÄISCHE PATENTSCHRIFT**

(45) Veröffentlichungstag der Patentschrift: **29.04.81**

(21) Anmeldenummer: **78101022.8**

(22) Anmeldetag: **29.09.78**

(51) Int. Cl.³: **C 07 C 45/29, B 01 J 8/06, C 07 C 47/04**

(54) Verfahren zur Herstellung von Formaldehyd.

(30) Priorität: **24.10.77 DE 2747581**

(43) Veröffentlichungstag der Anmeldung:
**02.05.79 Patentblatt 79/9**

(45) Bekanntmachung des Hinweises auf die Patenterteilung:
**29.04.81 Patentblatt 81/17**

(84) Benannte Vertragsstaaten:
**BE DE FR GB NL SE**

(56) Entgegenhaltungen:
**DE - A - 2 324 066
US - A - 2 412 014
US - A - 2 467 993**

(73) Patentinhaber: **BASF Aktiengesellschaft
Carl-Bosch-Strasse 38
D-6700 Ludwigshafen (DE)**

(72) Erfinder: **Aicher, Albrecht
Sonnenstrasse 21
D-6710 Frankenthal 6 (DE)**
Erfinder: **Flory, Klaus, Dr.
Im Schilling 2
D-6906 Leimen (DE)**
Erfinder: **Petri, Norbert, Dr.
Max-Beckmann-Strasse 17
D-6710 Frankenthal (DE)**
Erfinder: **Schuchart, Peter, Dr.
Bensheimer Ring 13b
D-6710 Frankenthal (DE)**
Erfinder: **Sebastian, Robert
Katharinenstrasse 4
D-6705 Deidesheim (DE)**

Courier Press, Leamington Spa, England.

Verfahren zur Herstellung von Formaldehyd

Die Erfindung betrifft ein Verfahren zur Herstellung von Formaldehyd durch Oxidation von Methanol in Gegenwart eines Metalloxidkatalysators bei erhöhter Temperatur, wobei Verdampfung des Methanols, Vermischung des Methanols mit der zu ihm im Gegenstrom geführten Luft und Umsetzung in derselben Reaktionszone unter bestimmten Bedingungen bezüglich Reaktionsbeginn, Mengenverhältnis und Vermischungszeit der Reaktionspartner durchgeführt werden.

Es ist aus Ullmanns Encyklopädie der technischen Chemie (4. Auflage), Band 11, Seiten 693 bis 696 bekannt, daß man Methanol mit Luft bei erhöhter Temperatur an Metalloxidkatalysatoren, in der Regel an Gemischen von Eisenoxid und Molybdänoxid, zu Formaldehyd oxidiert (Formox-Verfahren). In der Regel vermischt man zuerst vorgeheizte Luft mit Methanol, verdampft das Methanol in einem Verdampfer und leitet das Gemisch in einen Festbettröhrenreaktor, der den Katalysator enthält. Die Reaktion im Reaktor ist exotherm. Die freiwerdende Wärme wird einerseits über Wärmeträger, z.B. Mineralöl, die durch den Mantelraum des Reaktors gepumpt werden, aus dem Reaktor herausgeführt. Andererseits verläßt mit den austretenden Reaktionsgasen (Temperaturen von ca. 300°C) eine entsprechende Wärmemenge den Reaktor. Die von Wärmeüberträger bzw. von den Reaktionsgasen aufgenommene Reaktionswärmen dienen (loc. cit., Seite 693) der Erzeugung von Dampf bzw. zur Vorerwärmung der Prozeßluft im Vorwärmer.

In einer Arbeit in Chemie-Ingenieur-Technik, Band 31, 1959, Seiten 761 bis 765, wird ein Oxidationsverfahren beschrieben, bei dem die Luft zunächst in einem Wärmeaustauscher durch Abkühlung der heißen Reaktorgase vorgewärmt wird und dann in den Methanolverdampfer eintritt. Im Verdampfer wird flüssiges Methanol durch Abkühlung der vorgewärmten Luft verdampft. Das Methanol-Luft-Gemisch erhitzt man dann in einem weiteren Wärmeaustauscher ebenfalls durch Abkühlung der Reaktionsgase auf, bevor es in den Reaktor eintritt.

In der US-Patentschrift 2 812 309 ist ein Verfahren beschrieben, bei dem flüssiges Methanol in einem mit Dampf betriebenen Verdampfer verdampft und anschließend in einer Mischkammer mit der im Wärmeaustausch mit den heißen Reaktionsgasen vorgewärmten Luft vermischt wird.

Chemie Ingenieur-Technik (loc. cit., Seite 761 bis 762) weist auf die obere (über 36 Volumenprozent) und untere (unter 6, 7 bis 8 Volumenprozent) Explosionsgrenze des Methanolgehaltes in Luft/Methanolgemischen hin. Der Weg vom Ort der Methanolverdampfung bis zum Reaktor ist relativ lang. So können sich, bei Nichtbeachtung der Explosionsgrenzen, größere Volumina mit explosiblem Methanol-Luft-Gemisch füllen; entsprechend aufwendige Regel- und Steuerungseinrichtungen sind notwendig. Alle vorgenannten Verfahren zeigen die Vermischung von Methanoldampf und Luft im Gleichstrom und benötigen zusätzliche Wärmeüberträger für Verdampfung und/oder Erwärmung der Luft.

Die US—Patentschriften 2 467 993 und 2 412 014 lehren Verfahren, bei denen zuerst ein Gemisch von Ausgangsstoff, Luft und Stickstoffdioxiden hergestellt und dieses Gemisch dann über den Katalysator geführt wird. Als Ausgangsstoff verwendet man nicht Methanol sondern Methan. Man benötigt 2 Katalysatorschichten, von denen eine Metall und die andere Kieselsäure enthält. Der Reaktor kann mehrere Rohre enthalten, in denen sich die Reaktion abspielt.

Das in DE—OS 23 24 066 beschriebene Verfahren hat den Katalysator nicht auf viele Röhren verteilt, sondern der Katalysator ist nur in einer Schicht enthalten und wird nicht gekühlt. Das einströmende Gasgemisch enthält nur 2 bis 3 Vol.% Methanol gegenüber üblicherweise 6 bis 6,5 Vol.%, was zu erheblichem Mehraufwand führt, da große Volumina zu befördern sind.

Die Temperatur des Katalysators beträgt 200 bis 400°C. Dementsprechend sind die Temperaturen des den Katalysator verlassenden Formaldehyds und des wieder im Katalysator aufgeheizten Formaldehyds. Wenn man bedenkt, daß die eintretende Gasmenge bis zu 5mal so viel Formaldehyd gegenüber Methanol enthält, muß mit einer Zersetzung des Formaldehyds gerechnet werden (Beginn der Zersetzung bei 300°C, Jones u. Fowlie, J. appl. Chem. (1953), Seite 208). Auch Formaldehyd besitzt Explosionsgrenzen: 7 bis 72 Vol.% (Formaldehyde, J. F. Walker, Reinhold Publishing Corporation, 1964, Seite 39), wenn bis zu 5 mal so viel Formaldehyd wie Methanol im eintretenden Gas enthalten ist, so sind das bis zu 15 Vol.% Formaldehyd. Mit dieser und zusätzlich der Menge von 2—3 Vol.% Methanol ist man im Explosionsbereich des Formaldehyds. Es wird ausdrücklich beschrieben, daß Reaktoren mit zahlreichen Rohrbündeln keine guten Ergebnisse liefern. Das erfindungsgemäße Verfahren zeigt überraschend, daß eine sichere, nicht durch Explosion gefährdete Arbeitsweise infolge der erfindungsgemäßen Parameter erzielt werden kann. Insbesondere zeigt die deutsche Offenlegungsschrift weder einen Betrieb im Gegenstrom noch die Konstruktion und Dimension der erfindungsgemäßen Apparatur, ebenfalls nicht die Dimensionen der Einzelrohre, die, entgegen der Lehre der deutschen Offenlegungsschrift, in speziellen Bündeln angeordnet sind. Weiterhin war es überraschend, daß die Vorteile des erfindungsgemäßen Verfahrens, trotz anderer Bauart der Apparatur, beachtlich sind. Auch konnten der deutschen Offenlegungsschrift Parameter wie die Kombination einer röhrenförmigen Reaktionszone, die zahlreiche Rohre bestimmter Dimension aufweist, die Verdampfung des Methanols und seine Führung im Gegenstrom der Luft, die Dimensionen des Vermischungsraumes, die erforderlichen Dimensionen der Rohre, die Reihenfolge der Zusätze (Erhitzen, Luftleitung durch das Reaktionsrohr, Methanoldampf im Gegenstrom; Strömungsgeschwindigkeiten von Luft bzw. Methanoldampf) nicht entnommen werden.

Es wurde nun gefunden daß man Formaldehyd durch Oxidation von Methanol in Gegenwart eines Metalloxidkatalysators bei einer Temperatur von 200 bis 400°C mit Hilfe von Wärmeübertragung vorteilhaft erhält, wenn man die Umsetzung in einer röhrenförmigen Reaktionszone, die mindestens ein mit Katalysator gefülltes Reaktionsrohr von 10 bis 50 mm lichter Weite und mindestens ein der Methanolverdampfung dienendes Verdampfungsrohr von 10 bis 50 mm lichter Weite, Wärmeübertragungsmittel und eine mit den Reaktionsrohren über einen, ein Volumen von 25 bis 150 Kubikzentimeter je Quadratzentimeter Gesamtkatalysatorbettquerschnitt umfassenden Vermischungsraum verbundene Luftzuführung enthält, durchführt, wobei man zuerst die Reaktionszone auf eine Temperatur von 200 bis 400°C erhitzt, dann Luft durch die Reaktionsrohre leitet, nun Methanol durch die Verdampfungsrohre führt und darin verfdampft, anschließend den aus den Verdampfungsrohren im Gegenstrom zur mit einer Strömungsgeschwindigkeit von 0,01 bis 800 Kilogramm pro Minute zugeführten Luft mit einer Strömungsgeschwindigkeit von 0,001 bis 85 Kilogramm pro Minute austretenden Methanoldampf von einer Temperatur von 150 bis 350°C mit der Luft vermischt und schließlich das Luft-Dampfgemisch in den Reaktionsrohren umsetzt.

Im Hinblick auf den Stand der Technik liefert das Verfahren nach der Erfindung überraschend auf einfachere und wirtschaftlichere Weise Formaldehyd in guter Ausbeute, besserer Raum-Zeit-Ausbeute und in guter Reinheit. Die bei der Reaktion freiwerdende Wärme wird auf kürzestem Wege unmittelbar zur Verdampfung an das Methanol übertragen. Ein gesonderter Verdampfer entfällt. Der apparative Aufwand und somit die Investitionskosten sind niedriger als bei den herkömmlichen Verfahren. Energieverluste infolge mehrfacher Wärmeübertragung werden vermieden. Methanoldampf und Luft werden erst unmittelbar vor der Reaktion zusammengeführt. Hierdurch ist das Volumen, welches sich möglicherweise mit explosiblem Methanol-Luft-Gemisch füllen kann, kleiner als bei den bisher bekannten Verfahren. Die zur Sicherung der Anlage erforderlichen Druckentlastungsflächen können erheblich kleiner als bei den bisherigen Verfahrensweisen gehalten werden. Die Führung des Methanoldampfes im Gegenstrom läßt vergleichsweise nur jeweils geringe Anteile von Methanol auf große Mengen Luft treffen die Einstellung, Regelung und Kontrolle der unteren Explosionsgrenze ist somit wesentlich einfacher und betriebssicherer, wobei zusätzlich durch den sehr kleinen Vermischungsraum das Gemisch nach der Vermischung vorteilhaft rasch zum Katalysator gelangt. Der Wärmeüberträger erwärmt gleichzeitig den Katalysator und die Luft über dem Vermischungsraum, läßt das Methanol verdampfen und wirkt somit vor Beginn der Reaktion als Aufheizungskreislauf, nach Beginn der Reaktion als Kühlmittel und gleichzeitig Wärmeüberträger auf Verdampfung und Lufterwärmung; die Verdampfung ist selbstregelnd. Hat die Reaktion am Katalysator eingesetzt, so enthalten die austretenden Reaktionsgase nur noch den Anteil der Reaktionswärme, der die zur Erhitzung des Wärmeüberträgers und der Luft und damit zur Verdampfung des Methanols dienende Wärmemenge übersteigt. Infolge des kleinen Vermischungsvolumens wird ein homogenes Methanol-Luft-Gemisch für einen großen Belastungsbereich erzielt. Die Umsetzung kann mit höherer Methanol-konzentration im Methanol/Luft-Gemisch durchgeführt werden, die Strömungsgeschwindigkeit in den mit Katalysator gefüllten Rohren ist vergleichsweise geringer und damit der Katalysatorabrieb wesentlich vermindert.

Im Hinblick auf die US—Patentschriften 2 467 993 und 2 412 014 ist das erfindungsgemäße Verfahren überraschend einfacher und wirtschaftlicher. Weder werden 2 Katalysatorschichten noch die Verwendung von Stickoxiden benötigt. Ebenfalls unterscheidet sich der erfindungsgemäße Reaktor mit seinen Verdampfungs- und Reaktionszonen von den bekannten Reaktoren, die keine Verdampfungsrohre enthalten. Im Hinblick auf die deutsche Offenlegungsschrift ist das erfindungsgemäße Verfahren einfacher und wirtschaftlicher und zeigt die vorgenannten überraschenden Vorteile.

Alle diese vorteilhaften Ergebnisse des erfindungsgemäßen Verfahrens sind im Hinblick auf den Stand der Technik überraschend.

Für das Verfahren geeignete Ausgangsstoffe sind reines Methanol oder technisches Methanol. Auch Rohmethanol, das gegebenenfalls nach den in der DE—B—12 77 834, DE—C—1 235 881 und DE—C—1 136 318 beschriebenen Verfahren durch Abtrennung einer niedriger siedenden Fraktion bzw. durch Behandlung mit Oxidationsmitteln und/oder Alkalien gereinigt wird, kann verwendet werden.

Als oxidierendes Agens lassen sich sowohl der reine Sauerstoff als auch freien Sauerstoff enthaltende Gase, insbesondere Luft, verwenden. Sauerstoff und Methanol werden zweckmäßig im Molverhältnis von 1 bis 4,6, insbesondere von 1,5 bis 3 Mol Sauerstoff, insbesondere in Gestalt von Luft, je Mol Methanol angewandt.

Für die Umsetzung in den mit Metalloxiden als Katalysatoren gefüllten Reaktionsrohren können die bekannten Verfahren unter den entsprechenden Bedingungen (Ullmann, loc. cit. sowie die zum Stand der Technik aufgeführten Verfahren) durchgeführt werden. Bevorzugt gelangen Oxide des Eisens und des Molybdäns, ohne Trägermaterialien oder zweckmäßig auf einem Träger, zur Anwendung. Man läßt vorteilhaft 0,1 bis 1,6 Kilogramm, vorzugsweise 0,2 bis 1,1 Kilogramme Methanol je Stunde und Liter Katalysator in Gegenwart des Katalysators reagieren.

Der Katalysator kann von 3 bis 20, vorzugsweise von 4 bis 15 Gewichtsprozent Oxide des Eisens und des Molybdän, bezogen auf einen Träger, vorteilhaft einen Silikatträger, enthalten. In der Regel verwendet man die Oxide in einem Verhältnis voneinem Gewichtsteil $Fe_2O_3$ zu 3 bis 15 Gewichtsteilen $MoO_3$, vorzugsweise voneinem Gewichtsteil $Fe_2O_3$ zu 5 bis 7 Gewichtsteilen $MoO_3$. Gegebenenfalls können noch Kobalt-, Nickel-, Chrom-, Wolfram-, Aluminium-oxid, Phosphortri- bzw. Phosphorpentoxid, zweckmäßig in einer Menge von 0,5 bis 5, vorzugsweise 0,5 bis 3 Gewichtsprozent, bezogen auf den Träger, oder im Falle von trägerlosen Katalysatoren ien einer Menge von 0,1 bis 2 Gewichtsprozent, bezogen auf die Gesamtmenge an Oxid, im Katalysatro anwesend sein. Die Verbindungen der vorgenannten Elemente im Katalysator werden hier ohne Berücksichtigung ihrer tatsächlichen Konstitution als Oxide bezeichnet und berechnet. Es wird angenommen, daß im allgemeinen in solchen Fällen ein Gemenge von Eisenmolybdat, Molybdaten und den genannten Oxiden vorliegt.

Als Träger sind Silikatträger bevorzugt. Als Silikatträger werden im allgemeinen Alkali-, Erdalkali-silikate, insbesondere Calciumsilikat, Magnesiumsilikat, Zinksilikat, Cersilikat, Zirkonsilikat, Aluminiumsilikat oder ihre Gemische verwendet. Die Silikate können auch als Alumosilikate, Borosilikate oder Zeolithe in Frage kommen, bevorzugt sind entsprechende Magnesiumsilikate. Gegebenenfalls enthalten sie Zusätze wie Alkalisulfate bzw. Alkalioxide, z.B. Sodagläser, Sulfatgläser, Kaligläser, Kalisulfatgläser. Auch Silikate mit verschiedenen Kationen im Gemisch sind geeignet, z.B. in Gestalt von Silikaten des Typs von Olivin, Phenakit, Titanit, Chrysolit, Benitoit, Axinit, Milarit, Steatit, Orthoklas, Plagioklas, Albit.

Es können aber auch andere Trägermaterialien, z.B. Quarz, Tonerde, Siliciumcarbid, Porzellan, Magnesiumoxid oder Rutil, verwendet werden. Die Trägermaterialien können in kristallinem, amorphem oder gesintertem Zustand angewandt werden. Auch geschmolzenes Aluminiumoxid ist gut geeignet. Vorteilhaft kommen Träger in Betracht, die eine innere Oberfläche zwischen 0 und 5 $m^2$/g aufweisen. Man kann sie in Form von Körnern, Pillen, Ringen und vorteilhaft von Kugeln mit einem Durchmesser von 3 bis 12 Millimetern verwenden.

Der Katalysator besteht aus Teilchen beliebiger, aber vorzugsweise kugeliger Form, mit einem Durchmesser von 4 bis 12, vorzugsweise von 5 bis 10 Millimetern und einer Oberfläche von unterhalb 10, vorzugsweise von 0,01 bis 5 $m^2$ je g Katalysator. Im allgemeinen verwendet man einen kugeligen Silikatträger, der mit einer wäßrigen Lösung von Molybdän- und Eisensalzen behandelt wird. Bezüglich der Katalysatorherstellung wird auf vorgenannte Veröffentlichungen verwiesen.

Man kann ein oder zweckmäßig mehrere, vorzugsweise von 3 000 bis 22 000, insbesondere von 5 000 bis 16 000 Reaktionsrohre und/oder ein oder zweckmäßig mehrere, vorzugsweise 50 bis 1 500, insbesondere 150 bis 800 Verdampfungsrohre verwenden. Die Rohre können beliebig, aber in der Regel parallel zur Wand der Reaktionszone, innerhalb der Zone angeordnet sein. Die Rohre können zwar untereinander in Konstruktion, Material und/oder den Dimensionen verschiedenartig ausgestalte sein, vorteilhaft sind sie aber untereinander gleich. Der Querschnitt jedes Rohres kann beliebige Formen besitzen, zweckmäßig ovale und vorzugsweise runde Form. Zweckmäßig wählt man ein Längenverhältnis von Breite zu Länge der Rohrquerschnitte von 1 bis 3, vorzugsweise von 1 bis 1,5 für die Verdampfungsrohre und die Reaktionsrohre. Zwar können die Rohrquerschnitte innerhalb des Rohres untereinander bzw. vom Rohreingangsquerschnitt bzw. vom Rohrausgangsschlitzquerschnitt unterschiedlich sein, vorteilhaft wird man aber denselben Rohrquerschnitt in Form und Dimension von Eingang bis Ausgang 1 beibehalten. Bevorzugt sind sowohl als Verdampfungsrohre wie Reaktionsrohre Rohre von 50 bis 500, vorzugsweise von 100 bis 360 Zentimeter Rohrlänge, von 15 bis 40, vorzugsweise 15 bis 30 Millimeter lichter Weite. Als innere lichte Weite wird hier der reine Abstand von Rohrwand zu Rohrwand, d.h. der doppelte innere Radius kreisförmiger Rohre bzw. der doppelte Rohrradius abzüglich der doppelten Dicke des Rohrmantels, verstanden.

Die Reaktionszone ist bevorzugt vertikal angeordnet. Die Luft wird so von oben eingeführt und die Abgase von unten abgezogen. Man kann die Reaktionszone aber auch horizontal oder geneigt anrodnen, bei diesen Bauarten gelten sinngemäß alle Angaben der Beschreibung bezüglich oben (unten) im Sinne von der Luftzuführung zugewandt (dem abgeführten Reaktionsgemisch zugewandt), z.B. im Falle horozontaler Reaktionszonen im Sinne vorn (hinten). Vorteilhaft ist die Reaktionszone eine großes Rohr bzw. zweckmäßig eine Kammer mit kreisförmigem Querschnitt. Sie enthält die Temperaturzone und den darüberliegenden Vermischungsraum und mündet unten in den Austrittsraum des Reaktionsgemisches. Die Temperaturzone enthält die Rohre und das Wärmeübertragungsmittel. Der Vermischungsraum wird zweckmäßig durch den vorteilhalt senkrecht zur Wand der Reaktionszone und zu den Rohren liegenden, ebenen Boden, der lediglich durch die Ausgänge der Verdampfungsrohre und die Eingänge der Reaktionsrohre unterbrochen wird, und die Wände und Decke, die zweckmäßig als Haube oder Dom ausgestaltet sind, begrenzt. Eine bevorzugte Ausführungsform ist kegelförmig und domförmig, mit einem Winkel von 30 bis 60 Grad zum Vermischungsraumboden, wobei der Kegelmantel des kegelförmigen Domes die nach oben spitz zulaufende Wand des Raumes bildet und die Domspitze als Eingang der Luft (Luftzuführung) ausgebildet ist. Vorteilhaft ist der Lufteingang kreisförmig, hat einen Eingangsquerschnitt von 600 bis 9 000, insbesondere 800 bis 7 000 Quadratzentimetern, liegt paralle zum Boden des Vermischungsraumes und hat einen senkrechten Abstand von 50 bis 180, insbesondere von 60 bis 150 Zentimetern vom Boden des Vermischungsraumes. Der Vermischungsraum hat ein Volumen von 25 bis 150 Kubikzentimeter, insbesondere von 50 bis 140 Kubikzentrimeter je Quadratzentimeter Gesamtkalalysatorbettquerschnitt. Unter Gesamtkatalysatorbettquerschnitt wird die Summe der Querschnittsflächen aller mit Katalysator gefüllten Reaktionsrohre verstanden. Ein Verhältnis von 50 bis 300, insbesondere 100 bis 280 Kubikzentimeter Vermischungsraum je Gramm pro Minute aus den Verdampfungsrohren austretender Methanoldampf ist bevorzugt.

Unter dem Vermischungsraum liegt die Temperaturzone der Reaktionszone, die oben durch den Boden des Vermischungsraumes und unten durch einen zweckmäßig ähnlichen, ebenen und zur Wand der Reaktionszone senkrechten Boden, der ebenfalls duch die Rohre unterbrochen wird, begrenzt ist. Sie enthält die Rohre sowie eine Zuführung und Ableitung des Wärmeübertragungsmittels. Die Zuführung (Ableitung) kann beliebig, zweckmäßig im unteren (oberen) Abschnitt der Wand der Temperaturzone (Kammer- bzw. Rohrmantel der Reaktionszone) liegen. Der gesamte verbleibende Raum der Temperaturzone ist vorteilhaft mit Wärmeübertragungsmittel gefüllt, das so alle Rohre umspült; eine laufende Umpumpung des Wärmeübertragungsmittels, gerade um seinen Wärmeinhalt z.B. für weitere Verdampfungs- und Synthesezwecke auszunutzen, ist zweckmäßig, vorteilhaft mit 300 bis 4 500, insbesondere 500 bis 3 000 Kubikmeter pro Stunde Umpumpungsgeschwindigkeit. Das Verhältnis des Gesamtvolumens (ohne Rohrmantel) aller Rohre zum Volumen des Wärmeübertragungsmittels in der Temperaturzone beträgt vorteilhaft von 0,5 bis 1, insbesondere von 0,65 bis 0,85 zu 1. Die Verdampferrohre und die Reaktionsrohre können in beliebiger Weise, zweckmäßig jeweils in Serien gleichmäßig über die Temperaturzone verteilt, angeordnet sind. Bevorzugt stehen alle Rohre parallel zur Wand und senkrecht zum Boden des Vermischungsraumes und zum Boden der Temperaturzone. In einer bevorzugten Ausführungsform entspricht die Anrodnung jeder Serie von Rohren in der Temperaturzone und in den Böden im Querschnitt einem Polygon (Vieleck), zweckmäßig so angeordnet, daß alle Ecken des Polygons auf einem Kreis liegen und die Seiten gleich sind. Bevorzugt sind von 2 bis 20, insbesondere von 4 bis 10 polygonische Verdampfungsserien (Verdampfungskreise) und von 4 bis 90, insbesondere von 10 bis 60 polygonische Reaktionsrohrserien (Katalysatorkreise), wobei zweckmäßig der innerste Kreis ein Verdampfungskreis, der außerste Kreis ein Katalysatorkreis ist. Von innen nach außen wechseln vorteilhaft Verdampferkreise mit Katalysatorkreisen ab, wobei zweckmäßig zwischen 2 Verdampferkreisen ein bis 50 Katalysatorkreise liegen können. Bevorzugt sind die Abstände von Kreis zu Kreis gleich, der Durchmesser des innersten Kreises beträgt bevorzugt 0,1 bis 5 Prozent der Länge der gesamten lichten Weite der Temperaturzone und 50 bis 200 Prozent der Länge des Abstandes zwischen 2 Kreisen. Vorteilhaft enthält ein Kreis um so mehr Rohre, je weiter er vom Zentrum der Temperaturzone entfernt ist. Zweckmäßig hat der innerste Verdampferkreis von 2 bis 30, insbesondere von 6 bis 18 Rohre und jeder folgende Verdampferkreis von 1 000 bis 10, insbesondere von 800 bis 15 Prozent mehr Rohre als der vorhergehende engere Verdampferkreis und/oder der innerste Katalysatorkreis von 2 bis 30, insbesondere von 6 bis 18 Rohre und jeder folgende Katalysatorkreis von 500 bis 1, insbesondere von 100 bis 5 Prozent mehr Rohre als der vorhergehende engere Katalysatorkreis. Zweckmäßig treten die einzelnen Verdampferrohre durch den Boden des Vermischungsraumes und den Boden der Temperaturzone durch, wobei im Vermischungsraum und im Raum unterhalb der Temperaturzone alle Rohre eines Verdampferkreises mittels eines Kreisrohres verbunden sind. Die Verdampferrohre können auch frei in den Vermischungsraum hineinragen und von unterschiedlicher Länge sein. Die unteren Kreisrohre sind zweckmäßig jeweils über ein Verbindungsrohr mit der Methanolzuführung verbunden, die oberen Kreisrohre sind vorteilhaft an der Oberseite perforiert, so daß der Methanoldampf im Gegenstrom zur Luft austreten kann.

Unter der Temperaturzone und damit unter der Reaktionszone liegt der Ausgangsraum des abgeführten Reaktionsgemisches. Vorteilhaft ist er ebenfalls in Struktur und Dimensionen wie der Vermischungsraum ausgestaltet, d.h. bevorzugt kegel- und domförmig, zweckmäßig mit den vorgenannten bevorzugten Dimensionen des Vermischungsraumes. Das aus den Reaktionsrohren austretende Reaktionsgemisch umspült und erhitzt so vorteilhaft schon die Verbindungsrohre zu den Verdampfungskreisen und die Kreisrohre dieser Kreise.

Als Wärmeübertragungsmittel verwendet man zweckmäßig Diphenyl, chlorierte Diphenyle, Gemische von Diphenyl und Diphenyloxid, Mineralöle mit einem Siedepunkt zwischen 300 und 400°C oder Salzschmelzen, z.B. Natrium- und/oder Kaliumnitrat-Natriumnitrit-Gemische, gegebenenfalls zusammen mit Chromaten als Stabilisator, oder ein Gemisch von 47% KOH und 53% NaOH. Die Temperatur des Mittels wird in der Regel bei 250 bis 400°C, vorzugsweise von 280 bis 370°C, gehalten. Daher wird vorteilhaft das Wärmeübertragungsmittel solange bei diesen Temperaturen durch Erhitzen mit äußeren Wärmequellen gehalten, bis die Wärme der Reaktion diese Temperatureinstellung übernimmt.

Die Reaktion kann wie folgt durchgeführt werden: Man erhitzt das Wärmeübertragungsmittel und damit die Reaktionszone, insbesondere die Temperaturzone auf eine Temperatur von 200 bis 400°C, insbesondere von 250 bis 350°C, dann leitet man Luft mit einer Strömungsgeschwindigkeit von 0,01 bis 800, insbesondere von 0,02 bis 700 Kilogramm pro Minute und von einer Temperatur von 10 bis 300, insbesondere von 50 bis 250°C über Luftzuführung und Vermischungsraum durch die Reaktionsrohre hindurch, wobei die Temperatur bei 200 bis 400, insbesondere bei 250 bis 350°C gehalten wird. Dann wird flüssiges Methanol in solcher Menge über die Verbindungsrohre und die Verdampferkreise in die Verdampferrohre gegeben und in ihnen verdampft, daß sich eine Strömungsgeschwindigkeit von 0,001 bis 85, insbesondere von 0,002 bis 75 Kilogramm pro Minute austretendem Methanoldampf von einer Temperatur von 150 bis 350°C, vorzugsweise 200 bis 300°C, einstellt. Luft und Methanoldampf vermischen sich und werden zweckmäßig nach einer durchschnittlichen Verweilzeit des Methanoldampfes von 0,1 bis 1,5, insbesondere von 0,2 bis 1,0 Sekunden im Vermischungsraum in die Katalysatorrohre gefürt und dort umgesetzt. Ab Beginn der Umsetzung wird die Oxidationdes Methanols in der Regel bei einer Temperatur zwischen 280 und 400°C, vorzugsweise zwischen 290 und 340°C, drucklos oder unter Druck, durchgeführt. Das austretende Reaktionsgemisch wird über den Austrittsraum abgefürt. Aus dem Reaktionsgemisch trennt man in üblicher Weise, z.B. durch ein oder mehrere Wäschen des Gemisches mit Wasser im Gegenstrom, den Formaldehyd in Gestalt von 30- bis 60-gewichtsprozentigen Lösungen ab.

Der nach dem Verfahren der Erfindung herstellbare Formaldehyd ist Desinfektionsmittel, Gerbstoff, Reduktionsmittel und wertvoller Ausgangsstoff für die Herstellung von Kunstharzen, Klebmitteln, Kunststoffen und Hilfsmitteln in zahlreichen Industriesektoren. Bezüglich der Verwendung wird auf Ullmanns Encyklopädie der technischen Chemie, Band 7, Seite 670, verwiesen.

Die in den folgenden Beispielen angeführten Teile bedeuten Gewichtsteile.

Beispiel 1

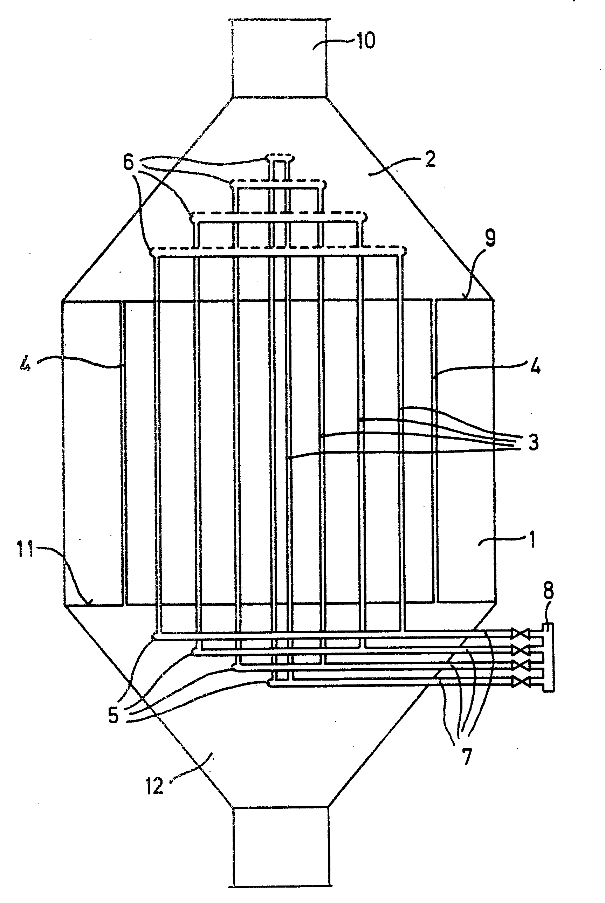

(Die angegebenen Zeichen beziehen sich auf Figur 1, die lediglich ein Anlageschema und damit das Grundprinzip des Verfahrens zeigt. Daher ist auch nur ein Teil der Rohre dargestellt).

Man verwendet einen vertikalen Reaktor mit einer Reaktionszone, die aus einer Temperaturzone (1) und einem Vermischungsraum (2) von 2,72 Kubikmeter besteht. Die Temperaturzone (1) enthält 4 Verdampferkreise (3) mit 294 Verdampfungsrohren und 40 Katalysatorkreise (4) mit 5 136 Reaktionsrohren. Verteilung der Verdampferrohre:

| 1. (innerster) Verdampferkreis | 6 Rohre (jeweils Rohrlänge 350 cm); |
|---|---|
| 2. (innerer) Verdampferkreis | 48 Rohre (jeweils Rohrlänge 320 cm); |
| 3. (äußeret) Verdampferkreis | 96 Rohre (jeweils Rohrlänge 280 cm); |
| 4. (äußerster) Verdampferkreis | 144 Rohre (jeweils Rohrlänge 250 cm); |

Die folgenden Zahlen bedeuten Katalysatorkreisnummern, wobei der innerste Katalysatorkreis 1 und der äußerste Katalysatorkreis 40 ist (in Klammern die Rohrzahlen):

1 (12); 2 (18); 3 (24); 4 (30); 5 (36); 6 (42); 7 (54); 8 (60); 9 (66); 10 (72); 11 (78); 12 (84); 13 (90); 14 (102); 15 (108); 16 (114); 17 (120); 18 (126); 19 (132); 20 (138); 21 (150); 22 (156); 23 (162); 24 (168); 25 (174); 26 (180); 27 (186); 28 (192); 29 (198); 30 (204); 31 (210); 32 (216); 33 (222); 34 (228); 35 (234); 36 (210); 37 (186); 38 (156); 39 (120); 40 (78).

Nach dem ersten Verdampferkreis folgen 6 Katalysatorkreise nach dem zweiten und dritten Verdampferkreis folgen 7 Katalysatorkreise, nach dem vierten Verdampferkreis folgen 20 Katalysatorkreis. Von allen Kreisen ist der innerste ein Verdampferkreis, der äußerste ein Katalysatorkreis. Die Abstände von Kreis zu Kreis betragen jeweils 3,2 Zentimeter, die Länge der Katalysatorrohre 200 Zentimeter, der Radius aller Katalysator- und Verdampferrohre 1,1 Zentimeter. Die Verdampferrohre endigen unten (5) und oben (6) in entsprechende Kreisrohre, die unten (5) über Verbindungsrohre (7) mit der Methanolzuführung (8) verbunden sind. Die oberen Kreisrohre (6) sind an der Oberseite perforiert. 120 Zentimeter über dem Boden (9) des kegelförmigen Vermischungsraumes (2) liegt die Luftzuführung (10). Unter dem Boden (11) der Temperaturzone (1) befindet sich der Austrittsraum (12). Als Wärmeübertragungsmittel werden 6 660 Teile Kaliumnitrat und 4 440 Teile Natriumnitrit in die Temperatursone (1) und als Katalysator 3 010 Teile eines Gemisches von 0,92 Gewichtsprozent Eisenoxid, 5,26 Gewichtsprozent Molybdänoxid und 0,19 Gewichtsprozent Chromoxid auf einem Träger aus Steatit in die Reaktionsrohre eingefüllt.

Nun wird die Reaktionszone auf eine Temperatur von 280°C erhitzt, dann Luft von 150°C mit einer Geschwindigkeit von 143 Kilogramm pro Minute durch den Vermischungsraum (2) und die Katalysatorkreise (4) geleitet und schließlich stündlich 603 Teile Methanol über Zuführung (8), Verbindungsrohre (7), untere Kreisrohre (5), Verdampferkreise (3) und obere Kreisrohre (6) der Luft im Gegenstrom entgegengeführt. Der Methanoldampf von 210°C tritt mit einer Strömungsgeschwindigkeit von 10,05 Kilogramm pro Minute aus, vermischt sich mit Luft und wird bei 300°C in den Katalysatorkreisen (4) umgesetzt. Das Reaktionsgemisch verläßt über den Austrittsraum (12) den Reaktor, wird auf 150°C abgekühlt und in einem Rieselturm in stündlich 237 Teilen Wasser gelöst.

Man erhält, bezogen auf stündlich zugeführte 603 Teile Methanol 543 Teile Formaldehyd (ber. 100%) pro Stunde in Gestalt einer 50-gewichtsprozentigen, wäßrigen Lösung mit einem Methanolgehalt von 0,1 Gewichtsprozent und einem Gehalt von 0,01 Gewichtsprozent Ameisensäure. Das entspricht einer Ausbeute von 96,1 Prozent der Theorie, bezogen auf eingesetztes Methanol. Die Ausbeute an Entstoff und der Methanolgehalt der anfallenden Formaldehydlösung bleiben während 480 Tagen konstant.

### Patentanspruch

Verfahren zur Herstellung von Formaldehyd durch Oxidation von Methanol in Gegenwart eines Metalloxidkatalysators bei einer Temperatur von 200 bis 400°C mit Hilfe von Wärmeübertragung, dadurch gekennzeichnet, daß man die Umsetzung in einer röhrenförmigen Reaktionszone, die mindestens ein mit Katalysator gefülltes Reaktionsrohr von 10 bis 50 mm lichter Weite und mindestens ein der Methanolverdampfung dienendes Verdampfungsrohr von 10 bis 50 mm lichter Weite, Wärmeübertragungsmittel und eine mit den Reaktionsrohren und Verdampfungsrohren über einen, ein Volumen von 25 bis 150 Kubikzentimeter je Quadratzentimeter Gesamtkatalysatorbettquerschnitt umfassenden Vermischungsraum verbundene Luftzuführung enthält, durchführt, wobei man zuerst die Reaktionszone auf eine Temperatur von 200 bis 400°C erhitzt, dann Luft durch die Reaktionsrohre leitet, nun Methanol durch die Verdampfungsrohre führt und darin verdampft, anschließend den aus den Verdampfungsrohren im Gegenstrom zur mit einer Strömungsgeschwindigkeit von 0,01 bis 800 Kilogramm pro Minute zugeführten Luft mit einer Strömungsgeschwindigkeit von 0,001 bis 85 Kilogramm pro Minute austretenden Methanoldampf von einer Temperatur von 150 bis 350°C mit der Luft vermischt und schließlich das Luft-Dampf-Gemisch in den Reaktionsrohren umsetzt.

### Claim

A process for preparing formaldehyde by oxidizing methanol over a metal oxide catalyst at from 200 to 400°C, with the aid of heat transfer, wherein the reaction is carried out in a tubular reaction zone which contains one or more catalyst-filled reaction tubes of from 10 to 50 mm internal width and one or more methanol vaporization tubes of from 10 to 50 mm internal width, a heat transfer medium and an air feed line connected to the reaction tubes and the vaporization tubes via a mixing chamber having a volume of from 25 to 150 cubic centimeters per square centimeter of total catalyst bed cross-section, and wherein the reaction zone is first heated to 200 — 400°C, air is then passed through the reaction tubes, thereafter methanol is passed through the vaporization tubes and vaporized therein, following which the methanol vapor, at 150 — 350°C, which issues at a flow rate of 0.001 — 85 kilograms per minute from the vaporization tubes in counter-current to air supplied at a flow rate of from 0.01 to 800 kilograms per minute is mixed with the said air, and finally the air-vapor mixture is reacted in the rreaction tubes.

### Revendication

Procédé pour la préparation de formaldéhyde par oxydation de méthanol en présence d'un catalyseur à base d'oxydes métalliques, à une température de 200 à 400°C au moyen d'un transfert de chaleur, caractérisé en ce qu'on mène la réaction dans une zone de réaction tubulaire qui comporte au moins un tube de réaction de 10 à 50 mm d'ouverture, rempli de catalyseur, et au moins un tube d'évaporation de 10 à 50 mm d'ouverture, servant à l'évaporation du méthanol, un agent de transfert de chaleur et une admission d'air raccordée aux tubes de réaction et aux tubes d'évaporation par une chambre de mélange ayant un volume de 25 à 150 cm³ par cm² de la section totale du lit de catalyseur, en commençant par chauffer la zone de réaction à une température de 200 à 400°C, puis en faisant passer de l'air à travers les tubes de réaction, en faisant alors passer du méthanol à travers les tubes d'évaporation où ils s'évapore, à la suite de quoi on mélange à l'air la vapeur de méthanol qui, à une température de 150 à 350°C, sort des tubes d'évaporation, à une vitesse d'écoulement de 0,001 à 85 kg/mn, à contre-courant par rapport à l'air introduit à une vitesse d'écoulement de 0,01 à 800 kg/mn, et enfin on fait réagir le mélange air-vapeur dans les tubes de réaction.